# EUROPÄISCHE PATENTSCHRIFT

(11) **EP 4 117 600 B1**
(45) Veröffentlichungstag und Bekanntmachung des Hinweises auf die Patenterteilung: **20.05.2026**
(21) Anmeldenummer: 21712227.4
(22) Anmeldetag: 10.03.2021
(51) Int. Cl.: A61H 9/00, A61H 33/12, A61H 35/00, A61M 21/00, A61M 11/00, A61M 16/06, A61M 16/14, A61H 33/06, A61N 5/06

(54) **VERWENDUNG VON CANNABINOIDEN IN THERAPIE- UND WELLNESSGERÄTEN**
USE OF CANNABINOIDS IN THERAPY AND WELLNESS APPARATUSES
UTILISATION DES CANNABINOÏDES DANS LES ÉQUIPEMENTS DE THÉRAPIE ET DE BIEN-ÊTRE

(30) Priorität: 11.03.2020 CH 2762020
(43) Veröffentlichungstag der Anmeldung: 18.01.2023
(73) Patentinhaber: JK-Holding GmbH, 53578 Windhagen (DE)
(72) Erfinder: GERSTENMEIER, Jürgen, 56567 Neuwied (DE)
(74) Vertreter: IPrime Rentsch Kaelin AG
(86) Internationale Anmeldenummer: PCT/IB2021/051983
(87) Internationale Veröffentlichungsnummer: WO 2021/181296

(56) Entgegenhaltungen:
- WO-A1-2017/118980
- WO-A2-02/36063
- WO-A2-2004/100852
- WO-A2-2017/208072
- CA-A1- 2 668 217
- CN-A- 108 992 340
- US-A1- 2018 318 529
- US-B1- 9 440 041
- US-B2- 10 299 515

## Beschreibung

Die vorliegende Erfindung betrifft eine Vorrichtung zur Beaufschlagung eines menschlichen Körpers mit Energie, mäss den Oberbegriffen der unabhängigen Patentansprüche.

Die vorliegende Erfindung betrifft weiter die Verwendung von Dampfgemischen, umfassend mindestens ein Cannabinoid in Vorrichtungen zur Beaufschlagung eines menschlichen Körpers mit Energie, ebenfalls gemäss Oberbegriffen der unabhängigen Patentansprüche.

### Technologischer Hintergrund

Cannabinoide und ihre synthetischen Analoga sind pharmazeutisch wirksame Sekundärstoffe, die hauptsächlich aus Extrakten der Hanfpflanze (Familie Cannabaceae) erhältlich sind. Die meisten Cannabinoide sind mehr oder weniger psychotrope Substanzen, welche auf das zentrale Nervensystem wirken und unter anderem mit analgetischen Effekten in Verbindung gebracht werden. Weitverbreitet ist inzwischen eine medizinische Akzeptanz einer Verwendung von Cannabinoid umfassenden Arzneidrogen, welche zur Linderung neuropathischer und entzündungsbedingter Schmerzen eingesetzt werden. Zahlreiche Studien weisen auf andere mögliche Anwendungsgebiete hin, welche gegenwärtig rege untersucht werden; so sollen Cannabinoide unter anderem zur Linderung der folgenden Symptome infrage kommen: Linderung spastischer Beschwerden, Verbesserung der Darmmotilität, antiemetische Effekte, Senkung des intraokularen Drucks, sowie zahlreiche unterstützende Effekte bei der Behandlung psychischer und psychosomatischer Erkrankungen, wie z.B. Angststörungen.

Hinsichtlich dieser therapeutischen Einsatzmöglichkeiten findet vermehrt die Zulassung von Arzneimitteln auf Cannabisbasis statt. In den meisten Ländern sind Cannabinoide Verschreibungspflichtig, wobei in der medizinischen Praxis die Verwendung vor allem für die Behandlung von chronischen Schmerzzuständen, wie z.B. bei neuropathischen oder durch Krebs verursachten Schmerzen, bei Spastik und Krämpfen, wie z.B. durch Multiple Sklerose oder andere neurologische Krankheiten ausgelöst werden können, oder bei Übelkeit und Appetitverlust in Verbindung mit Chemotherapien freigegeben wird. Neben der medizinischen Verwendung findet durch die fortschreitende Liberalisierung des Konsums von Cannabisprodukten mit vermindertem Tetrahydrocannabinolgehalt als Genussmittel eine zunehmende Akzeptanz von Cannabinoiden im Allgemeinen in der westlichen Gesellschaft statt.

Die Nutzung von Wellenessgeräten ist verbreitet zur Begleitung therapeutischer Massnahmen, welche für den Patienten unter Umständen mit starken Nebenwirkungen verbunden sind. Ebenso werden diese Geräte zur Linderung von Beschwerden eingesetzt, die auf Spannungszustände zurückzuführen sind, oder wenn mindestens ein positiver Effekt durch entspannungsfördernde Massnahmen erreichbar ist. So können z.B. Massagen und Lichttherapien Verspannungen lösen, die Durchblutung verbessern und durch positive psychosomatische Beeinflussung einen Entspannungsprozess in die Wege leiten, der einer Genesung förderlich ist.

Geeignete Vorrichtungen können z.B. Lichttherapiegeräte oder Solarien sein, welche dosierte Lichtmengen und/oder UV-Energie abgeben, um z.B. beim Patienten saisonale Verstimmungen aufgrund des verminderten Sonnenlichts zu beheben. Ebenso können mit Massagegeräten, wie z.B. Trockenmassagegeräten, die mittels eines geschlossenen Wasserstrahls gezielt Körperstellen massieren können, verspannungs- und krampflösende Therapien gezielt unterstützt werden.

Es besteht somit ein Bedarf nach derartigen Therapie- oder Wellnessgeräten, welche geeignet sind, unterstützend einerseits psychosomatische Beschwerden zu lindern oder therapeutische Massnahmen zu unterstützen.

CA2668217 A1 offenbart eine Vorrichtung zur Beaufschlagung eines menschlichen Körpers mit Energie. US10299515B2 offenbart einen Vaporisator für pflanzenbasierte Wirkstoffe, unter anderem auch für Cannabinoide.

### Darstellung der Erfindung

Es ist somit eine Aufgabe der Erfindung, mindestens einen Nachteil des Bekannten zu überwinden.

Es ist insbesondere eine Aufgabe der Erfindung, eine Vorrichtung eingangs genannter Art bereitzustellen, welche geeignet ist, eine therapeutische Behandlung und/oder eine Wellnesstherapie durchzuführen. Dabei soll die Vorrichtung vorzugsweise sicher in der Anwendung sein und eine genaue Dosierung der Behandlungsparameter ermöglichen.

Die Erfindung wird in den Patentansprüchen definiert.

Ein Aspekt der vorliegenden Erfindung betrifft eine Vorrichtung zur Beaufschlagung eines menschlichen Körpers mit Energie. Die Vorrichtung umfasst eine Kontaktfläche, welche einen Beaufschlagungsbereich definiert. In diesem Beaufschlagungsbereich ist ein menschlicher Körper so platzierbar, dass er in den Wirkbereich der Energie kommt. Die Vorrichtung umfasst weiter mindestens ein Mittel zur Abgabe der im Wesentlichen auf den Beaufschlagungsbereich gerichteten Energie. Weiter umfasst die Vorrichtung mindestens ein Mittel zur Erzeugung eines Dampfgemisches umfassend mindestens ein Cannabinoid.

Weiter umfasst die erfindungsgemässe Vorrichtung mindestens ein Mittel, um das Dampfgemisch mit einer Effektivdosis mindestens eines Cannabinoids ins Umfeld der Atemöffnungen des menschlichen Körpers zu führen.

Im Sinne der vorliegenden Erfindung können als geeignete Cannabinoide sowohl solche verstanden werden, die natürlich aus der genannten Pflanzenfamilie der Cannabisgewächse, insbesondere Cannabis sativa oder Cannabis indica, extrahiert werden können, als auch durch Isomerisation entsprechender Vorstufen, z.B. von Cannabidiol zu Tetrahydrocannabinol, als auch die synthetisch hergestellte Analoga verstanden werden. Das bekannteste Cannabinoid ist Δ⁹-Tetrahydrocannabinol, welches durch Extraktion aus Hanfpflanzen gewonnen werden kann. Extraktionsverfahren sind dem Fachmann bekannt.

Im Sinne der vorliegenden Erfindung kann mit dem Umfeld der Atemöffnung des menschlichen Körpers, z.B. der Kopfbereich des menschlichen Körpers, gemeint sein, nämlich das Umfeld der Nase und des Mundes.

Im Sinne der vorliegenden Erfindung kann eine Kontaktfläche eine im Wesentlichen horizontal ausgestaltete Liegefläche sein. Ebenfalls denkbar ist eine Kontaktfläche, welche eine Sitz- oder Lehnfläche umfasst, in welcher sich der entsprechende menschliche Körper setzen und/oder legen kann. In einer besonderen Ausführung ist die Kontaktfläche eine Bodenplatte oder Stehfläche, auf der sich ein Mensch stehend im Beaufschlagungsbereich platzieren kann, dass er in den Wirkbereich der Energie kommt.

Im Sinne der vorliegenden Erfindung kann die Effektivdosis als diejenige Dosis eines Wirkstoffs im Dampfgemisch, insbesondere eines Cannabinoids, verstanden werden welche bei einem statistisch relevanten Anteil an Menschen einen gewünschten pharmakologischen Effekt erzielt. Der gewünschte pharmakologische Effekt kann dabei vom Fachmann in Abhängigkeit des zu erzielenden Behandlungserfolgs bestimmt werden. So kann die Effektivdosis z.B. bei als Genussmitteln im Umfang von Wellnesstherapien verwendeten erfindungsgemässen Vorrichtungen entsprechend tief angesetzt werden, sodass z.B. lediglich eine leichte Muskelentspannung oder ein angenehmes Wohlgefühl erreicht werden soll. Bei medizinisch-therapeutischen Anwendungen kann die Effektivdosis im Hinblick auf die zu erzielende therapeutische Absicht gewählt werden. So kann z.B. die Effektivdosis in Abhängigkeit der pharmakologischen Wirkung für verschiedene Cannabinoide unterschiedlich ausfallen. Die entsprechenden Effektivdosen sind in den gesetzlichen, und in der Pharmakopöe vorgeschriebenen Richtlinien enthalten (siehe unter anderem Pharmacopoea Helvetica oder Pharmacopoea Europaea). Für das am besten untersuchte Cannabinoid, z.B. THC, sind Effektivdosen im Bereich von zwischen 5 mg bis 60 mg pro Tag angegeben, welche in Tablettenform täglich verabreicht werden. Bei flüssig verabreichten, z.B. für die Behandlung von Glaukomen eingesetzten Arzneimitteln wird z.B. eine Konzentration in Lösung von zwischen 1 bis 7 % THC für eine einmalige Tagesbehandlung vorgesehen.

In einer besonderen Ausführungsform ist die Effektivdosis einstellbar auf der Basis eines Verdampfungsmittels auf Ölbasis mit einem THC Gehalt von zwischen 0.1 und 1 Vol.% und einem Cannabidiol von zwischen 10 und 20 Vol.%.

In einer besonderen Ausführungsform ist die Effektivdosis einstellbar über die Cannabinoid Menge des Verdampfungsmittels, insbesondere wobei die Effektivdosis zwischen 36% und 61% des Cannabinoid Gehalts des Verdampfungsmittels im Dampfgemisch aufweist. Im Betrieb würde zum Beispiel der Anteil des Cannabinoids aus dem Verdampfungsmittel der im Dampfgemisch als Effektivdosis zur Verfügung steht von einem Fachmann auf der Basis der Temperatur und der Art und Weise wie das Dampfgemisch erzeugt wird, z.B. konduktiv, konvektiv und/oder mittels Ultraschalls, einstellbar sein.

In einer besonderen Ausführungsform der vorliegenden Erfindung ist das Umfeld der Atemöffnung des menschlichen Körpers ein definiertes Umfeld. Dieses definierte Umfeld kann z.B. ein bestimmtes Volumen umfassen. Es ist anhand des definierten Volumens dann möglich, für einen Anwender die entsprechende Effektivdosis im Hinblick den gewünschten Effekt einzustellen. So kann z.B. eine bestimmte Atmosphäre mit einem Dampfgemisch geschaffen werden, welche sicherstellt, dass durch Inhalation des Dampfgemisches im Umfeld der Atemöffnung die entsprechende Effektivdosis erzielt werden kann. Dies kann die Sicherheit der erfindungsgemässen Vorrichtungen erhöhen, und insbesondere unerwünschte Nebenwirkungen, zum Beispiel psychotrope Effekte verhindern.

Mit der Lösung der vorliegenden Erfindung ist eine eingangs genannte Vorrichtung bereitgestellt, welche therapeutisch sicher ist und synergistisch die positiven Effekte einer Beaufschlagung mit Energie mit einer Behandlung mit Arzneidrogen verbindet, welche den therapeutischen und/oder Wellnesseffekt optimal unterstützen können, insbesondere mit einem Dampfgemisch umfassend eine Effektivdosis eines Cannabinoids. Insbesondere durch das Schaffen eines definierten Umfelds der Atemöffnungen kann eine sichere Verabreichung der entsprechenden Effektivdosis stattfinden, welche verhindert, dass eine zu hohe Dosierung z.B. zu unerwünschten psychotropen Nebeneffekten führt, oder eine zu geringe Dosierung nicht den gewünschten therapeutischen Effekt erzielt.

In einer besonderen Ausführungsform ist das Mittel zur Abgabe der im Wesentlichen auf den Beaufschlagungsbereich gerichteten Energie ausgelegt, um elektromagnetische Energie abzugeben. Besonders bevorzugt handelt es sich bei der elektromagnetischen Energie um Strahlungsenergie. Ganz besonders bevorzugt handelt es sich bei der Strahlungsenergie um UV-Strahlung.

In einer besonders bevorzugten Ausführungsform ist das Mittel zur Abgabe der im Wesentlichen auf den Beaufschlagungsbereich gerichteten Energie ein Leuchtmittel. Das Leuchtmittel kann ausgestattet sein, um ein bestimmtes Farbspektrum wiederzugeben. Dazu kann das Leuchtmittel mit entsprechenden Filtern ausgestattet sein.

Besonders bevorzugt ist das Leuchtmittel in der Lage, Licht im sichtbaren Spektrum abzugeben. Dieses Spektrum kann den Bereich von zwischen ca. 400 nm und bis ca. 1100 nm umfassen, insbesondere den Bereich von zwischen ca. 400 nm und bis ca. 750 nm umfassen.

In einer besonders bevorzugten Ausführungsform umfasst das Leuchtmittel Filter, welche schädliche UV-Strahlung absorbieren und/oder reflektieren. Insbesondere UV-Strahlung im Bereich von unter 380 nm kann gesundheitlich schädlich sein.

Besonders bevorzugt ist das Leuchtmittel ausgestaltet, um UV-Strahlung im Bereich von zwischen 200 und 380 nm zu reflektieren und/oder absorbieren.

In einer besonders bevorzugten Ausführungsform ist das Leuchtmittel eine Solariumlampe. Solarien werden weitgehend als Wellnessgeräte verwendet, welche z.B. eine Hautbräunung aus kosmetischen Gründen erzielen sollen, oder durch die Beaufschlagung mit UV-Licht positive Wellnesseffekte erzielen sollen. In der Medizin werden Solarien verwendet, um bestimmte Hautkrankheiten zu behandeln.

In einer besonderen Ausführungsform ist die Kontaktfläche eine Liegefläche einer Solarium-Anlage zur liegenden Beaufschlagung mit UV-Licht.

In einer weiteren besonderen Ausführungsform ist die Kontaktfläche eine Stehfläche eines Stehsolariums zur stehenden Beaufschlagung mit UV-Licht.

In einer besonders bevorzugten Ausführungsform ist das Leuchtmittel eine UV-Strahlenquelle auf LED-Basis.

Solarien können nachweislich jahreszeitlich bedingte Verstimmungen therapieren, indem durch Lichtmangel erzeugte Verstimmungen gelindert oder ganz behoben werden. Diese Therapie kann zusätzlich durch die erfindungsgemässe Vorrichtung und die Erzeugung einer Effektivdosis eines Cannabinoids im Umfeld der Atemöffnungen während der Behandlung verbessert werden. Dadurch kann z.B. die gesamte Therapiedauer reduziert werden, was insgesamt helfen kann die Menge an Strahlung auf den Körper zu reduzieren.

In einer besonderen Ausführungsform ist das Mittel zur Abgabe der im Wesentlichen auf den Beaufschlagungsbereich gerichteten Energie ausgelegt, um kinetische Energie abzugeben. Besonders bevorzugt ist das Mittel ausgelegt, um einen gerichteten Fluidstrahl abzugeben.

In einer besonderen Ausführungsform ist das Mittel zur Abgabe der im Wesentlichen auf den Beaufschlagungsbereich gerichteten Energie eine Strahldüse, welche ausgelegt ist, einen Fluidstrahl, insbesondere einen Wasserstrahl auf den Beaufschlagungsbereich abzugeben. Solche Strahldüsen werden unter anderem verwendet, um gezielt und dennoch schonend muskuläre Verspannungen zu beheben. Dazu wird z.B. ein gerichteter Wasserstrahl einer definierten Intensität mittels einer Düse auf ein Körperteil gerichtet. Ebenfalls bekannt sind Vorrichtungen, welche die Behandlung mit Wasserstrahlen in einem trockenen Zustand ermöglichen.

Die Vorrichtung zur trockenen Massage mittels Wasserstrahlen (DE 20 2019 105 636 U1) zeigt eine solche Trockenmassagevorrichtung, bei der eine wasserfeste Hülle so ausgelegt ist, dass ein Körper im Wirkbereich der Energie durch den Wasserstrahl nicht nass wird; mit anderen Worten, das Wasser ist in durch eine wasserundurchlässige Membran hermetisch zurückgehalten.

In einer besonderen Ausführungsform der vorliegenden Erfindung ist das Mittel zur Erzeugung eines Dampfgemisches ein Vaporisator.

Im Sinne der vorliegenden Erfindung kann unter einem Vaporisator ein Gerät zur Verdampfung von Wirkstoffen verstanden werden. Dabei können die Wirkstoffe schonend, also z.B. gerade soweit erwärmt werden, dass sie verdampfen. Ein Oxidieren der Wirkstoffe kann somit verhindert werden. Vaporisatoren für den Konsum von Genussmitteln wie Tabak, Cannabis oder anderen Kräutern bekannt. Aus der Medizin sind ebenfalls Vaporisatoren bekannt, z.B. im Bereich der Anästhesie, um bestimmte Effektivdosen eines anästhetischen Wirkstoffs ins Umfeld der Atemöffnung eines menschlichen Körpers zu erzeugen und zu führen. Zur Erzeugung des Dampfgemisches kann eine Zusammensetzung verwendet werden, welche eine vorbestimmte Menge des Wirkstoffs enthält. Durch die Einstellung der Betriebsparameter für die Verdampfung und die Rückkopplung, z.B. mit einem definierten Umfeld der Atemöffnungen des menschlichen Körpers, kann eine entsprechende Effektivdosis eingestellt werden. Die Zusammensetzungen können Hilfsstoffe enthalten, wie z.B. Propylenglykol oder Glyzerin zusätzlich zu den Wirkstoffen. Auch können erfindungsgemäss Nutzbare Zusammensetzungen zusätzlich zum Cannabinoid weitere zur Verdampfung geeignete Arzneidrogen umfassen, insbesondere eine Arzneidroge ausgewählt aus der Gruppe bestehend aus: Afrikanisches Löwenohr (Leonotis leonurus), Ayahuasca, Yajé (Banisteriopsis caapi), Baldrian (Valeriana officinalis), Blauer Lotus (Nymphaea caerulea), Damiana (Turnera diffusa, Eukalyptus (Eucalyptus globulus), Fliegenpilz (Amanita muscaria), Hopfen (Humulus lupulus), Isländisches Moos (Cetraria islandica), Johanniskraut (Hypericum perforatum), Kamille (Chamomilla recutita), Kratom (Mitragyna speciosa), Lavendel (Lavandula sp.), Passionsblume (Passiflora incarnata), Pfefferminze (Mentha × piperita), Salbei (Salvia officinalis), Schafgarbe (Achillea spp.), Sinicuichi (Heimia salicifolia), Steppenraute (Peganum harmala), Thymian (Thymus vulgaris), Wahrsagesalbei (Salvia divinorum), Yohimbe (Pausinystalia yohimbe), und Zitronenmelisse (Melissa officinalis).

Besonders bevorzugt umfasst die Zusammensetzung das Cannabinoid Δ⁹-Tetrahydrocannabinol (C₂₁H₃₀O₂). Geeignete Zusammensetzungen können zum Beispiel mit pharmazeutisch erhältlichem Dronabinol mit THC Mengen von zwischen 1 und 2 Vol.% erhalten werden.

In einer besonderen Ausführungsform der vorliegenden Erfindung umfasst das Mittel, um das Dampfgemisch mit einer Effektivdosis mindestens eines Cannabinoids ins Umfeld der Atemöffnung des menschlichen Körpers zu führen, ein Rückhaltemittel, besonders bevorzugt eine Rückhaltehaube.

Die Rückhaltehaube ist vorzugsweise geeignet, eine Effektivdosis mindestens eines Cannabinoids im Umfeld der Atemöffnungen des menschlichen Körpers in einem Dampfgemisch zu erzeugen und zu erhalten. Besonders bevorzugt ist die Rückhaltehaube mit einem definierten Volumen ausgestaltet. Ganz besonders bevorzugt ist die Rückhaltehaube derart im Wirkbereich der Energie angeordnet, dass die Atemöffnungen des menschlichen Körpers vollständig in der Rückhaltehaube untergebracht sind. Die Rückhaltehaube schliesst in einer bevorzugten Ausführungsform nicht vollständig, sodass ein Gasaustausch mit der Umgebung stattfinden kann. Die Rückhaltehaube ist vorzugsweise so ausgestaltet, dass sich eine Atmosphäre mit dem Dampfgemisch rund um die Atemöffnungen des menschlichen Körpers bilden kann, aber dergestalt, dass durch Ausatmen des menschlichen Körpers durch die Nase die Abluft des Ausatmens ausserhalb der Rückhaltehaube geführt wird. Besonders bevorzugt ist die Rückhaltehaube in Bezug auf den menschlichen Kopf im Scheitelbereich geschlossen und im Kinnbereich offen, sodass ein Ausatmen die ausgeatmete Luft ausserhalb der Rückhaltehaube führt.

In einer besonderen Ausführungsform ist die Rückhaltehaube im Inneren beschichtet. Eine mögliche Beschichtung wäre eine Nanotextur, z.B. eine Nanotextur zur Erzeugung eines Lotuseffektes, welche ein Beschlagen der Rückhaltehaube verhindert.

Eine derart ausgestaltete Rückhaltehaube hätte vorteilhafterweise keinen nachteiligen Effekt auf Strahlungsenergie, welche dann durch die Rückhaltehaube hindurch auf den Körper beaufschlagt wird. Eine solche Rückhaltehaube könnte z.B. in einem Solarium Verwendung finden, ohne dass dabei der Bräunungseffekt auf den Kopf des Benutzers eingeschränkt würde.

Es wurde zudem überraschend festgestellt, dass ungeachtet der chemischen Struktur der Cannabinoide insbesondere des THC ((-)-Δ⁹-trans-Tetrahydrocannabinol) durch eine allfällige UV-Strahlung kein Zerfall der Cannabinoide innerhalb eines Wirkzeitraums im Dampfgemisch stattfindet.

Geeignete Wirkzeiträume können zwischen 1 min und 30 min, insbesondere zwischen 5 und 15 min, ganz besonders bevorzugt ca. 8 min betragen.

In einer besonderen Ausführungsform ist die Rückhaltehaube im Wesentlichen durchlässig für elektromagnetische Wellen ausgestaltet. Besonders bevorzugt ist sie durchlässig für elektromagnetische Wellen in den Wellenlängenbereichen zwischen 380 nm und 1100 nm, weiter insbesondere für zwischen 380 nm und 750 nm. Zu diesem Zweck kann die Rückhaltehaube z.B. aus UV-durchlässigem Polymethylmethacrylat (Plexiglas) hergestellt sein. Besonders bevorzugt sind mindestens die Bereiche, welche im Beaufschlagungsbereich zwischen dem menschlichen Körper und dem Mittel zur Abgabe der im Wesentlichen auf den Beaufschlagungsbereich gerichteten Energie zum Liegen kommen aus einem für elektromagnetische Wellen im Wesentlichen durchlässigen Material ausgestaltet.

In einer besonderen Ausführungsform ist die Rückhaltehaube integraler Bestandteil der Vorrichtung zur Beaufschlagung eines menschlichen Körpers mit Energie. So kann die Rückhaltehaube zum Beispiel einem Solarium Bestandteil eines Deckelelmentes sein. Ein Deckelelement kann ein beweglich ausgebildeter Teil eines Solariums sein, welcher gemeinsam mit mindestens einer Kontaktfläche ein Umfeld der Atemöffnungen des menschlichen Körpers und den Beaufschlagungsbereich definiert. Bei Stehsolarien können zwei Deckelelemente vorgesehen sein.

In einer besonderen Ausführungsform ist die Vorrichtung zur Beaufschlagung eines menschlichen Körpers mit Energie ein Solarium mit Liegefläche als Kontaktfläche und die Rückhaltehaube integraler Bestandteil eines Deckelelementes, so dass ein Volumen definiert wird von zwischen 450 dm³ und 1000 dm³, bevorzugt zwischen 580 dm³ und 800 dm³. Bevorzugt ist dieses Volumen das Umfeld der Atemöffnungen des menschlichen Körpers.

In einer besonderen Ausführungsform ist die Vorrichtung zur Beaufschlagung eines menschlichen Körpers mit Energie ein Solarium mit Stehfläche als Kontaktfläche und die Rückhaltehaube integraler Bestandteil mindestens zweier Deckelelemente, so dass ein Volumen definiert wird von zwischen 1000 dm³ und 2000 dm³, bevorzugt von ca. 1100 dm³. Bevorzugt ist dieses Volumen das Umfeld der Atemöffnungen des menschlichen Körpers.

Viele Solarienanlagen sind mit verschliessbar ausgestaltet, so dass ein Wirkbereich einer Beaufschlagung mit Licht betreten werden kann, und der Wirkbereich durch ein oder mehrere Deckelelemente in Verbindung mit der mindestens einen Kontaktfläche definiert wird. Diese Art von Solarienanlagen können derart verschliessbar ausgestaltet sein, dass ihr gesamtes Innenvolumen als Umfeld der Atemöffnungen des menschlichen Körpers angesehen werden können, und mit dem entsprechenden Dampfgemisch füllbar ist.

In einer besonderen Ausführungsform definiert die Rückhaltehaube ein Volumen von zwischen 200 dm³ und 400 dm³, bevorzugt von zwischen 250 dm³ und 350 dm³, besonders bevorzugt von ca. 275 dm³ auf. Bevorzugt ist dieses Volumen das Umfeld der Atemöffnungen des menschlichen Körpers.

Die Rückhaltehaube kann in einer weiteren Ausführungsform mit einem Drehlager ausgestattet sein, so dass sie von einem offenen auf einen geschlossenen Zustand überführbar ist.

In einer besonderen Ausführungsform ist die Rückhaltehaube ausgelegt, um ein Entweichen des Dampfgemisches aus dem Umfeld der Atemöffnungen des menschlichen Körpers im Wesentlichen zu verhindern. Zu diesem Zweck können Ansaugöffnen oder Gebläse an der Rückhaltehaube vorgesehen sein, die ein Austreten der Dämpfe zu verhindern, respektive austretendes Dampfgemisch abzusaugen in der Lage sind. Ebenfalls denkbar ist eine im Wesentlichen konische Ausgestaltung der Rückhaltehaube, bei der sich das Dampfgemisch aufgrund der im Vergleich zur Umgebungsluft vergleichsweise höheren Temperatur in einer Konus-Spitze sammelt. Nach erfolgter Behandlung kann die Rückhaltehaube zum Beispiel abgesaugt werden.

In einer besonderen Ausführungsform umfasst die erfindungsgemässe Vorrichtung einen Speicherbehälter für ein Verdampfungsmittel mit mindestens einem Cannabinoid.

In einer weiteren besonderen Ausführungsform umfasst die Vorrichtung ein Heizelement zur Beheizung einer definierten Menge eines Verdampfungsmittels, sodass ein Dampfgemisch mit einer definierten Menge des mindestens einen Cannabinoids entstehen kann.

In einer besonderen Ausführungsform kann das Heizelement so ausgestaltet sein, dass es direkt auf das Verdampfungsmittel einwirkt. Alternativ und/oder ergänzend kann das Heizelement so ausgestaltet sein, dass es indirekt auf das Verdampfungsmittel einwirkt.

In einer besonderen Ausführungsform ist das Heizelement zur konduktiven Beheizung eines Verdampfungsmittels ausgelegt.

In einer ergänzenden und/oder alternativen Ausführungsform ist das Heizelement für eine konvektive Beheizung des Verdampfungsmittels ausgestaltet.

In einer weiteren alternativen und/oder ergänzenden Ausführungsform findet das Erzeugen des Dampfgemischs mit einer definierten Menge des mindestens einen Cannabinoids ohne Erwärmung statt. Dies kann z.B. dadurch bewerkstelligt werden, dass ein Luftstrom mit einer gesättigten Atmosphäre mit dem geeigneten Cannabinoid vermischt wird. Alternativ und/oder ergänzend kann ein Ultraschallvernebler vorgesehen sein zur Erzeugung des Dampfgemisches.

In einer besonderen Ausführungsform ist das Verdampfungsmittel mit mindestens einem Cannabinoid eine Zusammensetzung auf einer zur inhalativen Applikationsform geeigneten festen oder flüssigen Basis, umfassend das mindestens eine Cannabinoid. Geeignete, auf fester oder flüssiger Basis bestehende Verdampfungsmittel sind bekannt. Das Verdampfen des Cannabinoids kann entweder direkt aus der flüssigen Phase geschehen oder über Sublimation direkt aus einer Basis.

In einer besonderen Ausführungsform ist das Heizelement ausgestaltet, um einen Temperaturbereich von zwischen 100° C und 235° C zur Erzeugung des Dampfgemisches zu erreichen. Besonders bevorzugt ist das Heizelement für einen Temperaturbereich von zwischen 165° C und 200° C, ganz besonders bevorzugt von ca. 200° C ausgestaltet.

In einer besonderen Ausführungsform umfasst das Verdampfungsmittel neben dem Wirkstoff, resp. der Arzneidroge, resp. dem Cannabinoid, weitere Hilfsstoffe.

In einer besonderen Ausführungsform handelt es sich bei diesen Hilfsstoffen um Hilfsstoffe, ausgewählt aus der Gruppe bestehend aus: Propylenglykol, Glyzerin, Wasser, Dexpanthenol, andere aromatische und/oder nicht aromatische Öle, Aromen oder die Viskosität der Verdampfungsmittel regulierende Stoffe.

In einer besonderen Ausführungsform umfasst die Vorrichtung mindestens einen Ventilator zur Erzeugung eines Luftstroms im Umfeld der Atemöffnungen des menschlichen Körpers. Besonders bevorzugt ist der Luftstrom zur Extraktion des Dampfgemisches mit einer Effektivdosis mindestens eines Cannabinoids aus einer Rückhaltehaube ausgestaltet. In dieser Ausführungsform kann der Ventilator zur Zufuhr von Frischluft im Umfeld der Atemöffnungen, insbesondere in der Rückhaltehaube dienen.

Alternativ und/oder ergänzend kann der Ventilator ausgestaltet sein, um nach Beendigung einer Therapie- und/oder Wellnesssitzung die Rückhaltehaube, resp. das Umfeld der Atemöffnungen vom Dampfgemisch mit dem mindestens einen Cannabinoid zu befreien. Ein derartiger Abzug kann sicherstellen, dass die Behandlungszeit nicht überschritten wird und eine Effektivdosis nur über einen bestimmten definierten Zeitraum verabreicht wird. Dieser Zeitraum kann besonders bevorzugt mit der Beaufschlagung mit Energie zeitlich abgestimmt sein. Somit kann eine Therapie- und/oder Wellnesssitzung entsprechend den optimalen Nutzen aus der Synergie zwischen der Beaufschlagung mit Energie, wie z.B. der Trockenmassage mit Wasserstrahl oder der Bestrahlung mit UV-Strahlen, und dem gewünschten therapeutischen Einsatz des Cannabinoids gekoppelt werden.

In einer besonderen Ausführungsform umfasst die erfindungsgemässe Vorrichtung mindestens einen optoelektronischen Sensor. Besonders bevorzugt ist dieser optoelektronische Sensor zur Ermittlung mindestens eines fotoelektrisch messbaren physiologischen Parameters des menschlichen Körpers im Wirkbereich der Energie ausgelegt. Zum Beispiel kann der optoelektronische Sensor ein Sensor zur optischen Pulsmessung sein. Alternativ und/oder ergänzend kann der optoelektronische Sensor auch ein UV-Sensor sein, der z.B. die Menge an aufgenommener UV-Strahlung oder die Intensität dieser UV-Strahlung misst.

In einer besonderen Ausführungsform können Sensoren zur Steuerung der Betriebsparameter der Vorrichtung ausgebildet sein. Dadurch liesse sich z.B. über einen Behandlungszeitraum eine Steuerung und Regelung der einzelnen Parameter der Vorrichtung erzielen. Zum Beispiel könnte in einer Trockenmassagevorrichtung, welche mit einer Rückhaltehaube und einer Vorrichtung zur Erzeugung eines Dampfgemisches mit einer Effektivdosis Cannabinoid ausgestatteten Anlage, der gewünschte therapeutische Effekt z.B. anhand einer bestimmten Zieldurchblutung des Oberflächengewebes ausgerichtet werden. Die entsprechende Effektivdosis wird variabel eingestellt, sodass sie in Einklang mit der Beaufschlagung mit kinetischer Energie durch die Massageliege geeignet ist, um einen entsprechenden Zielwert zu erreichen, der durch den optoelektronischen Sensor gemessen und über eine Steuerungsanlage durch direktes Ansteuern der Massagedüsen und/oder der Mittel, um ein Dampfgemisch im Umfeld der Atemöffnungen des menschlichen Körpers zu erzeugen, eingestellt wird.

In einer besonderen Ausführungsform umfasst die erfindungsgemässe Vorrichtung eine Steuerung, welche die Betriebsparameter der Mittel zur Erzeugung des Dampfgemisches und der Mittel zur Abgabe der im Wesentlichen auf den Beaufschlagungsbereich gerichteten Energie regelt und/oder steuert. Diese Steuerungseinheit kann zudem ausgestaltet sein, um Messdaten von Sensoren, wie z.B. dem optoelektronischen Sensor, wie oben beschrieben, zu empfangen, auszuwerten und in entsprechende Steuersignale umzuwandeln, welche einen gewünschten Effekt erzielen sollen.

In einer besonderen Ausführungsform umfasst die erfindungsgemässe Vorrichtung einen bioelektronischen Sensor. Besonders bevorzugt handelt es sich beim bioelektronischen Sensor um einen zum Messen eines Widerstands geeigneten Sensor. Derartige Sensoren sind bereits für die Messung von Körperwiderständen, z.B. für das Ermitteln von Atemfrequenzen und/oder von Körperfettanteilen, im Gebrauch.

In einer besonderen Ausführungsform umfasst das Mittel, um das Dampfgemisch mit einer Effektivdosis mindestens eines Cannabinoids im Umfeld der Atemöffnungen des menschlichen Körpers zu führen, eine Einlassöffnung, welche ausserhalb einer Rückhaltehaube erzeugtes Dampfgemisch in das Innere der Rückhaltehaube leitet.

In einer besonderen Ausführungsform ist die Rückhaltehaube im Wesentlichen schalenförmig ausgestaltet, sodass sie geeignet ist, um mindestens einen Teil des Kopfes und/oder Oberkörpers eines mit der erfindungsgemässen Vorrichtung behandelten menschlichen Körpers mindestens teilweise zu umschliessen. Besonders bevorzugt ist die Rückhaltehaube als Gefäss ausgebildet, welches geeignet ist, eine entsprechende Atmosphäre mit einer Effektivdosis mindestens eines Cannabinoids für einen bestimmten Zeitraum zurückzuhalten.

In einer besonderen Ausführungsform umfasst das Mittel, um das Dampfgemisch mit einer Effektivdosis mindestes eines Cannabinoids im Umfeld der Atemöffnungen des menschlichen Körpers zu führen, mindestens eine Auslassöffnung. Besonders bevorzugt ist die Auslassöffnung mit einer Ansaugvorrichtung verbunden. Die Auslassöffnung kann ausgebildet sein, um eine entsprechende Atmosphäre zu evakuieren. Dazu kann die Auslassöffnung auch mit einem Ventilator, wie weiter oben beschrieben, wirkverbunden sein, indem z.B. eine Schlauchverbindung zwischen der Auslassöffnung und dem Ventilator besteht.

Ein weiterer Aspekt der vorliegenden Erfindung betrifft ein Verfahren zum Betreiben einer beschriebenen Vorrichtung. Das Verfahren umfasst den Schritt des Bereitstellens einer Vorrichtung zur Beaufschlagung eines menschlichen Körpers. Besonders bevorzugt handelt es sich um eine Vorrichtung mit einer beliebigen, sich nicht gegenseitig ausschliessenden Kombination an Merkmalen der weiter oben geschilderten erfindungsgemässen Vorrichtung. Weiter wird ein Verdampfungsmittel mit mindestens einer verdampfbaren Arzneidroge bereitgestellt. Besonders bevorzugt ist die Arzneidroge ein Cannabinoid. Das Verfahren umfasst weiter den Schritt des Verdampfens des Verdampfungsmittels, sodass ein Dampfgemisch mit einer Effektivdosis der mindestens einen Arzneidroge entsteht. Besonders bevorzugt entsteht ein Dampfgemisch mit einer Effektivdosis mindestens eines Cannabinoids.

Das Dampfgemisch wird in das Umfeld der Atemöffnung eines menschlichen Körpers geführt. Dazu können z.B. entsprechende Schlauchverbindungen und/oder Einlassöffnungen vorgesehen sein, welche ein erzeugtes Dampfgemisch in den Teil des Beaufschlagungsbereichs leiten, in dem die Atemöffnungen des menschlichen Körpers angeordnet sind. Besonders bevorzugt wird das Dampfgemisch so in das Umfeld der Atemöffnungen eines menschlichen Körpers geführt, dass die Effektivdosis des mindestens einen Cannabinoids in einem Rückhaltemittel entsteht. Besonders bevorzugt handelt es sich bei dem Rückhaltemittel um eine Rückhaltehaube.

Das erfindungsgemässe Verfahren kann zudem den Schritt des Regulierens, Steuerns und/oder Anpassens des Verdampfens des Verdampfungsmittels an eine Beaufschlagungsroutine, welche durch die Vorrichtung erzeugt wird, umfassen.

Dazu kann im erfindungsgemässen Verfahren z.B. auch ein Messen oder ein Detektieren eines bestimmten Parameters vorgesehen sein. So kann z.B. ein gewisser physiologischer Zustand zur Regulierung einer Effektivdosis genutzt werden. In einer konkreten Ausführungsform könnte die Effektivdosis in einem bestimmten Bereich definiert werden, welche einen zulässigen Bereich darstellt, in der / dem ein gewisser physiologischer Wert zu erreichen ist. So kann z.B. eine Effektivdosis eines Cannabinoids in einem Dampfgemisch vorgesehen sein, welche zwischen 5 mg und 15 mg THC umfasst. Die exakte Menge in der Effektivdosis wird abhängig gemacht von einem gewissen physiologischen Parameter, der von den Sensoren gemessen und an die Steuerungseinheit gemeldet wird. Wird der physiologische Parameter erreicht, so wird von einer Erhöhung der Effektivdosis abgesehen. Die Vorrichtung kann in diesem konkreten Beispiel dazu ausgestaltet sein, die Effektivdosis schrittweise zu erhöhen. Im konkreten Beispiel könnte dies bedeuten, dass eine zur konvektiven Erwärmung eines Verdampfungsmittels ausgelegte Mittel solange und in dieser Form den Luftstrom an dem Verdampfungsmittel vorbeileiten, bis die gewünschte Effektivdosis erreicht wird, resp. bis der Sensor den zu erzielenden physiologischen Parameter an die Steuereinheit meldet. Um ein weiteres Erhöhen der Effektivdosis zu verhindern, kann z.B. ein Bypass vorgesehen sein, in dem der Luftstrom dann nicht weiter am Verdampfungsmittel vorbeigeführt wird und somit weiteres THC in das Umfeld der Atemwege gelangt, also im konkreten Beispiel in die Rückhaltehaube.

Ein weiterer Aspekt der vorliegenden Erfindung betrifft die Verwendung von Dampfgemischen umfassend mindestens ein Cannabinoid in Vorrichtungen zur Beaufschlagung eines menschlichen Körpers mit Energie. Dazu wird ein Dampfgemisch mit einer Effektivdosis mindestens eines Cannabinoids ins Umfeld der Atemöffnungen eines menschlichen Körpers geführt.

Es hat sich herausgestellt, dass ein synergistischer Effekt zwischen Vorrichtungen zur Beaufschlagung eines menschlichen Körpers und der Verwendung von Dampfgemischen mit mindestens einem Cannabinoid besteht. Insbesondere in Wellnesstherapien kann mit gering dosierten Cannabinoidkonzentrationen im Umfeld der Atemöffnungen des menschlichen Körpers eine Steigerung des Entspannungseffekts und des allgemeinen Wohlbefindens eines Menschen erreicht werden.

Die vorliegende Verwendung eignet sich zudem auch für den medizinisch-therapeutischen Bereich. Das Behandlungsspektrum kann von therapeutischen Verfahren zur Linderung von Nebenwirkungen und Schmerzen bis zur Verbesserung psychosomatischer Beschwerden reichen.

In einer besonderen Ausführungsform umfasst die erfindungsgemässe Verwendung die Verwendung von einer Effektivdosis mindestens eines Cannabinoids als Dampfgemisch in Verbindung mit einer Beaufschlagung eines Menschen mit Licht zur Linderung psychosomatischer Beschwerden und zur Steigerung des allgemeinen Wohlbefindens.

In einer weiteren Ausführungsform umfasst die erfindungsgemässe Verwendung das Verwenden eines Dampfgemisches umfassend mindestens ein Cannabinoid in Kombination mit einer Beaufschlagung des Patienten mit kinetischer Energie, z.B. mit einem Wasserstrahl einer Massagedüse, bevorzugt einer Trockenmassagevorrichtung der eingangs geschilderten Art, zur Linderung psychosomatischer Beschwerden und Steigerung des allgemeinen Wohlbefindens.

In einer besonderen Ausführungsform umfassen die psychosomatischen Beschwerden saisonale Verstimmungszustände, wie sie z.B. in den Wintermonaten vorkommen können. Es ist nachgewiesen, dass saisonale Verstimmungen in den Wintermonaten auf den Mangel an Sonnenlicht zurückzuführen sind. Es wurde überraschend festgestellt, dass Kombinationsbehandlungen mit einem Cannabinoid und der entsprechenden Beaufschlagung, z.B. mit ultraviolettem Licht, die psychosomatischen Beschwerden einer saisonalen Verstimmung effektiv und mit kurzen Therapiesitzungen massgeblich lindern können.

In einer besonderen Ausführungsform umfasst die erfindungsgemässe Verwendung eine Rückhaltehaube, welche geeignet ist, eine Effektivdosis des mindestens einen Cannabinoids im Umfeld der Atemöffnungen des menschlichen Körpers zu erzeugen und zu erhalten.

Im Sinne der vorliegenden Erfindung umfasst die Verwendung des mindestens einen Cannabinoids auch synthetische Analoga, wie z.B. Dimethylheptylpyran (DMHP).

In einer besonderen Ausführungsform umfasst die Verwendung ein Verdampfungsmittel mit einer bestimmten Konzentration eines Cannabinoids. Dieses Verdampfungsmittel kann auch mit pflanzlichen Extrakten aus der Cannabispflanze erzeugt werden. Alternativ und/oder ergänzend ist die direkte Verwendung von Teilen der Cannabispflanze ebenfalls geeignet, um ein Verdampfungsmittel mit dem entsprechenden Wirkstoffgehalt zu erzeugen. Dazu sind pharmakognostisch ermittelte und standardisierte Arzneidrogen mit Bestandteilen der Cannabispflanze besonders geeignet. So ist z.B. besonders feingemahlenes Pulver aus Blüten der Cannabispflanze (Cannabis flos) zur Verwendung geeignet.

Grundsätzlich sind für die erfindungsgemässe Verwendung nahezu alle exogenen Cannabinoide geeignet. Insgesamt sind bis zu 70 Cannabinoide bekannt. Die Effektivdosis kann nach dem gewünschten Effekt eingestellt werden. So ist bei einer niedrigen Dosierung ein analgetischer, unter Umständen sedativ-hypnotischer Effekt erzielbar. Bei einer Überdosierung oder einer sehr hohen Dosierung kann ein veränderter Bewusstseinszustand eintreten. Bekannte Effektivdosen sind dem Fachmann für die Behandlung von erhöhtem Augeninnendruck, Schmerzen, Blutdruck, Übelkeit, Appetitmangel oder auch als Appetitzügler bekannt.

Eine erfindungsgemässe Verwendung kann in einer besonderen Ausführungsform die Verwendung elektromagnetischer Energie umfassen, insbesondere Strahlungsenergie. In dieser Verwendung wird die Effektivdosis des mindestens einen Cannabinoids in Kombination mit einer Strahlungsbehandlung, besonders bevorzugt einer UV-Strahlungsbehandlung, verabreicht. Diese Kombination hat sich besonders vorteilhaft erwiesen zur Behandlung von saisonalen Gemütsschwankungen.

In einer weiteren besonderen Ausführungsform umfasst die erfindungsgemässe Verwendung die Verwendung kinetischer Energie. Besonders bevorzugt wird die kinetische Energie als gerichteter Fluidstrahl verwendet. Dies kann z.B. durch einen Wasserstrahl, welcher von einer Düse gezielt auf ein Körperteil des zu behandelnden Individuums gerichtet ist, bewerkstelligt werden. Solche Wassermassagegeräte sind bekannt. Besonders bevorzugt werden Wassermassagegeräte verwendet, bei denen man nicht nass wird, d.h. zur Trockenmassage geeignet sind. Solche verfügen über eine Membran, welche den Wasserstrahl zurückhält, die kinetische Energie aber bis zu einem gewissen Grad durch Verformen der Membran auf den menschlichen Körper zu übertragen in der Lage sind.

Es hat sich überraschend gezeigt, dass die Verbindung von solchen Massagegeräten mit der Verabreichung einer Effektivdosis eines Cannabinoids besonders geeignet ist, um muskuläre Verspannungen, Spannungszustände und durchblutungsbedingte Verhärtungen der Muskulatur zu lindern.

Für einen Fachmann versteht es sich von selbst, dass eine erfindungsgemässe Ausgestaltung der beschriebenen Vorrichtung, des entsprechenden Verfahrens und der entsprechenden Verwendung eine beliebige Kombination der genannten Merkmale verwirklichen kann, sofern sich diese nicht gegenseitig ausschliessen. Weitere vorteilhafte Merkmale werden im Folgenden für den Fachmann aus der detaillierten Beschreibung und den konkreten Ausführungsbeispielen erkenntlich.

Im Folgenden wird die vorliegende Erfindung anhand konkreter Figuren und Ausführungsbeispielen näher erläutert, ohne auf diese beschränkt zu sein.

### Figurenbeschrieb

Anhand der nachfolgenden Figuren werden Ausführungsbeispiele der Erfindung beschrieben. Es zeigen
- Fig. 1: eine erfindungsgemässe Vorrichtung;
- Fig. 2: die erfindungsgemässe Vorrichtung der Fig. 1 in der Anwendung;
- Fig. 3: eine weitere Ausführungsform der erfindungsgemässen Vorrichtung;
- Fig. 4: eine weitere Ausführungsform der erfindungsgemässen Vorrichtung, und
- Fig. 5: eine weitere Ausführungsform der erfindungsgemässen Vorrichtung.

### Ausführung der Erfindung

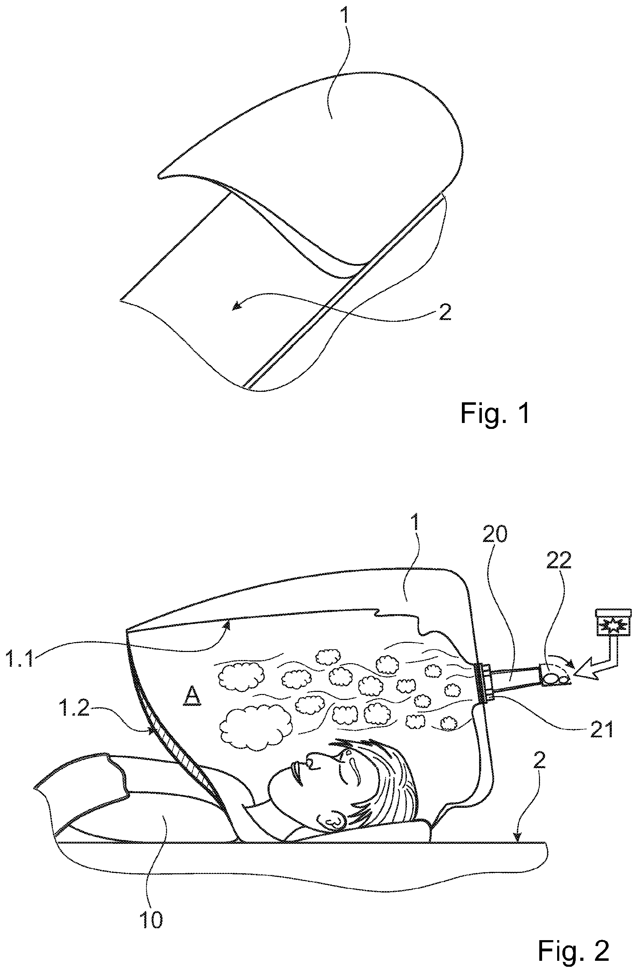

Die Fig. 1 zeigt eine besonders einfache Ausführungsform einer erfindungsgemässen Vorrichtung. An einer Kopfseite einer Kontaktfläche 2, welche im vorliegenden Beispiel als Liegefläche ausgestaltet ist, ist eine Rückhaltehaube 1 ausgestaltet.

Diese Rückhaltehaube 1 kann im vorliegenden Fall opak ausgestaltet sein, z.B. aus einem Kunststoff. Für die vorliegende Anwendung kann es vorteilhaft sein, wenn die Rückhaltehaube 1 den Kopfbereich zudem etwas abdunkelt. Die Rückhaltehaube 1 ist im Scheitelbereich geschlossen und öffnet sich nach unten zum Unterkörper hin. Die vorliegende Ausgestaltung ist in den Dimensionen so gewählt, dass der Kopf und die Schultern eines Menschen, welcher auf der Kontaktfläche 2 liegt, im Innern der Rückhaltehaube 1 sind, während die Gliedmassen, der Oberkörper ausserhalb der Rückhaltehaube zum Liegen kommen.

Die Kontaktfläche 2 kann im vorliegenden Beispiel als Membran ausgestaltet sein. Die Membran kann einen flüssigkeitsgefüllten Raum abdichten, in welchem Strahldüsen angeordnet sind, welche geeignet sind, einen Wasserstrahl auf die Kontaktfläche zu richten, sodass diese gleichzeitig einen Beaufschlagungsbereich definiert. Im Betrieb würde sich ein Benutzer auf die Kontaktfläche legen und durch diese mittels Wasserstrahlen mit kinetischer Energie beaufschlagt werden. Gleichzeitig würde sein Kopf unter der Rückhaltehaube 1 zum Liegen kommen. Die Rückhaltehaube 1 kann z.B. (im vorliegenden Beispiel nicht gezeigt) mit einer die Entspannung fördernden LED-Beleuchtung ausgestaltet sein. So können z.B. rote LED-Streifen im Innern oder am Rand der Rückhaltehaube eine gedämpfte, warme Atmosphäre schaffen, welche der Entspannung dienen kann.

Die Rückhaltehaube kann (ebenfalls nicht gezeigt) mit Einlassöffnungen versehen sein. Durch diese Einlassöffnungen kann ein Dampfgemisch mit einer Effektivdosis mindestens eines Cannabinoids geleitet werden. Liegt der Patient nun mit dem Kopf unter der Rückhaltehaube, so bildet sich eine kontrollierbare Atmosphäre rund um den Kopf, welcher die Administration einer bestimmten Effektivdosis eines Cannabinoids an den Patienten ermöglicht. Im vorliegenden Beispiel ist die Rückhaltehaube konvex ausgestaltet, sodass ein Volumen definiert wird, in dem das Dampfgemisch bestehen kann.

Mit der erfindungsgemässen Vorrichtung, wie sie in der Fig. 1 gezeigt ist, kann z.B. eine Wellnessanlage, welche zur Behandlung von psychosomatischen Beschwerden, wie z.B. saisonalen Verstimmungen oder von muskulären Verspannungen, Verwendung finden kann. Je nach geplanter Anwendung kann ein entsprechender Fachmann die Zusammensetzung eines Dampfgemisches definieren, und die entsprechende Effektivdosis festlegen, um einen gewünschten therapeutischen oder sedativen Effekt zu erzielen.

Dies ist näher erläutert am Beispiel der Fig. 2, welche die in der Fig. 1 gezeigte erfindungsgemässe Vorrichtung schematisch in Benutzung darstellt.

Ebenfalls ist eine Kontaktfläche 2 vorgesehen, auf welcher sich ein Patient 10 hinlegen kann. Über den Kopf des Patienten 10 ist eine Rückhaltehaube 1 platzierbar, welche ein Umfeld der Atemöffnungen A als Volumen definiert. Die Rückhaltehaube 1 ist auf der Innenseite 1.1 mit Leuchtdioden ausgestaltet, welche den Entspannungseffekt verbessern sollen. Ebenfalls denkbar ist eine Beschichtung der Rückhaltehaube 1, welcher verhindern soll, dass sich Wasserdampfkondensat im Innern der Rückhaltehaube bildet. Wasserdampfkondensat könnte zur Tropfenbildung führen, was im vorliegenden Beispiel für den Entspannungseffekt nicht gewünscht wäre. Ebenfalls denkbar wäre es, die Rückhaltehaube 1 mit Kühl- und/oder Heizelementen vorzusehen, entweder um eine entsprechende Atmosphäre zu schaffen oder um das Entstehen eines Kondensats zu verhindern. Die Rückhaltehaube 1 ist im distalen Bereich, d.h. nach zu den Extremitäten hin über eine Öffnung 1.2 belüftbar.

Ein Gasaustausch mit der Umgebung führt dazu, dass die Effektivdosis über die Zeit abnimmt, sofern kein Nachschub an Dampfgemisch erfolgt. Das Dampfgemisch wird über einen Zufuhrstutzen 20 in die Rückhaltehaube 1 geleitet. Das Dampfgemisch wird im vorliegenden Beispiel durch einen konvektiven Verdampfungsprozess erzeugt. Dazu wird ein Verdampfungsmittel 22 mit einem warmen Luftstrom in einer vordefinierten Temperatur umströmt, sodass die gewünschte Menge des Verdampfungsmittels 22 verdampft und über den Zufuhrstutzen 20 und die Auslassöffnung 21 in die Rückhaltehaube 1 geführt wird.

Obschon im vorliegenden Beispiel die Rückhaltehaube opak ausgestaltet ist, so kann sie selbstverständlich bei dieser Anwendung auch transparent ausgestaltet sein.

Im Betrieb würde ein Heizelement (nicht gezeigt) eine bestimmte Menge eines Verdampfungsmittels, z.B. ein Gemisch umfassend fein gemahlene 1.7 mg Cannabis mit auf eine Betriebstemperatur von zwischen 130 °Celsius und 226 °Celsius geheizter Luft derart umströmt, dass sich ein Dampfgemisch bildet. Besonders geeignet ist eine Temperatur von ca. 185 °Celsius. Das im vorliegenden Beispiel verwendete Cannabis weist einen Gehalt mindestens an Tetrahydrocannabinol (THC), Cannabidiol (CBD) und Cannabinol (CBN) auf. Der THC Gehalt beträgt zwischen 3.5 und 4.5%, bevorzugt 4.15% in standardisierter Mischung. CBD und CBN sind als Spuren vorhanden. Der Wassergehalt beträgt im vorliegenden Beispiel ca. 10 bis 15 Gew.% (s.a. Gieringer, D., et al.; Cannabis Vaporizer Combines Efficient Delivery of THC with Effective Suppression of Pyrolytic Compounds; Journal of Cannabis Therapeutics, October 2008).

Ein daraus resultierender Cannabis Dampf kann einen THC Gehalt von zwischen 36 und 61% aufweisen. Ausgehend von diesen Daten, kann der Fachmann auf der Basis eines Volumens im Umfeld der Atemöffnungen eines Patienten gerichtet eine Effektivdosis eines Dampfgemisches erzeugen.

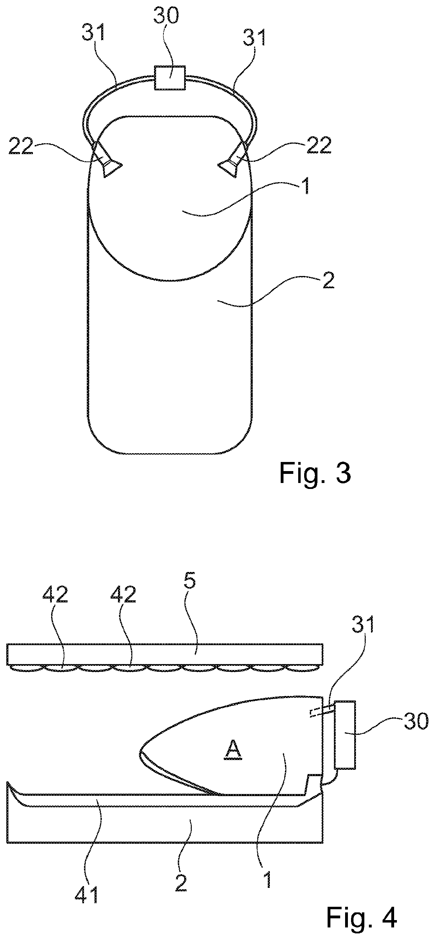

Die Fig. 3 zeigt eine erfindungsgemässe Vorrichtung mit einer Kontaktfläche 2 und einer entsprechenden ausgestalteten Rückhaltehaube 1 schematisch, wie sie mittels zweier Zufuhrstutzen 20, welche mit Schlauchverbindungen 31 an einen Vaporisator 30 angeschlossen sind, mit einem entsprechenden Dampfgemisch mit einer Effektivdosis eines Cannabinoids gespiesen werden können.

Eine entsprechende Ausführungsform für eine Bestrahlungsvorrichtung ist in der Fig. 4 gezeigt. Auch in der Fig. 4 ist eine Kontaktfläche ausgebildet, auf der sich ein Patient liegend hinlegen kann. Anders als in der vorangegangenen Ausführungsform ist die Kontaktfläche lediglich dazu da, den Patienten für die Beaufschlagung mit Energie konkret auszurichten. Die Kontaktfläche 2 sowie eine gegenüberliegende Deckelfläche 5 sind entsprechend mit Leuchtmitteln 42, 41 versehen, welche einen Beaufschlagungsbereich bilden, auf welchem der Patient mit Licht beaufschlagt wird. In einem Solarium wären dies entsprechend UV-Leuchten. In diesem Beaufschlagungsbereich erstreckt sich eine Rückhaltehaube 1, welche ein Umfeld für die Atemöffnungen des Patienten A definiert. Diese Rückhaltehaube 1 ist mittels einer Schlauchverbindung 31 mit einem Vaporisator verbunden. Im Vaporisator wird mit einer geeigneten Methode, z.B. mittels einer konduktiven Erhitzung eines Verdampfungsmittels ein entsprechendes Dampfgemisch erzeugt, welches über die Schlauchverbindung 31 in die Rückhaltehaube 1 geschleust wird. Im vorliegenden Beispiel ist die Rückhaltehaube selbstverständlich transparent ausgestaltet, zum Beispiel aus einem UV-durchlässigen Polymethylmethacrylat, wie sie Beispielsweise als Plexiglas unter dem Markennamen Alltop^{®} für Gewächshäuser verwendet werden. Ebenfalls besonders geeignet ist SUNACTIV GS 2458 Plexiglas.

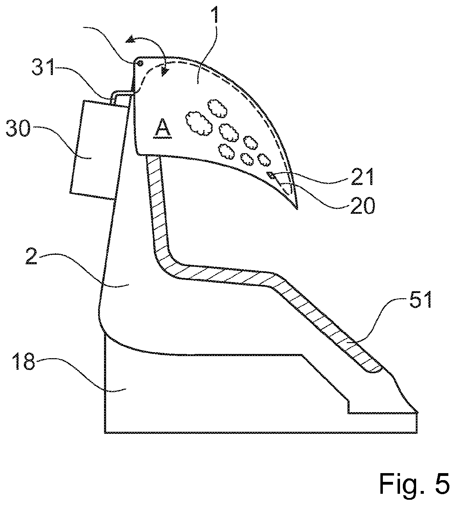

Eine alternative Ausführungsform ist in der Fig. 5 gezeigt, bei der die Vorrichtung für eine sitzende Anwendung gedacht ist.

Auch in dieser Vorrichtung handelt es sich bei der Energie um kinetische Energie, und die Beaufschlagung findet mittels Massageelementen statt.

Entsprechend ist die Kontaktfläche 2 mit einer entsprechenden mechanischen Massagevorrichtung 51, wie z.B. rotierender Massageknüppel versehen. Auch diese können in einer Membran eingebunden sein, um den Komfort während der Therapie zu erhöhen. Die Vorrichtung steht auf einem Sockel 18. Im Betrieb setzt sich der Patient auf die Massageknüppel 51, und eine in diesem Fall hochklappbare Rückhaltehaube 1 wird derart über den Kopf des Patienten geschlossen, dass das Umfeld der Atemöffnungen A ein definiertes Umfeld bildet. In dieses Umfeld A wird ein in einem Vaporisator 30 über Schlauchverbindungen 31 geleitetes Dampfgemisch mittels eines Zufuhrstutzens 20 und einem Vernebler 21 dem Patienten zugeführt.

## Patentansprüche

1. Vorrichtung zur Beaufschlagung eines menschlichen Körpers mit Energie, umfassend:
a. Eine **Kontaktfläche** (2), welche einen Beaufschlagungsbereich definiert in welchem ein menschlicher Körper in den Wirkbereich der Energie platzierbar ist;
b. Mindestens ein **Mittel zur Abgabe der im Wesentlichen auf den Beaufschlagungsbereich gerichteten Energie,** und
mindestens ein **Mittel zur Erzeugung eines Dampfgemisches** umfassend mindestens ein Cannabinoid, und
mindestens ein **Mittel um das Dampfgemisch mit einer Effektivdosis mindestens eines Cannabinoids ins Umfeld der Atemöffnungen (A) des menschlichen Körpers zu führen.**

2. Vorrichtung gemäss Anspruch 1, wobei das Mittel zur Abgabe der im Wesentlichen auf den Beaufschlagungsbereich gerichteten Energie ausgelegt ist um elektromagnetische Energie, insbesondere Strahlungsenergie abzugeben.

3. Vorrichtung gemäss Anspruch 1, wobei das Mittel zur Abgabe der im Wesentlichen auf den Beaufschlagungsbereich gerichteten Energie ausgelegt ist, um kinetische Energie abzugeben, insbesondere einen gerichteten Fluidstrahl abzugeben.

4. Vorrichtung gemäss einem der Ansprüche 1 bis 3, wobei das Mittel zur Erzeugung eines Dampfgemisches einen **Vaporisator** umfasst.

5. Vorrichtung gemäss einem der Ansprüche 1 bis 4, wobei **das Mittel um das Dampfgemisch mit einer Effektivdosis mindestens eines Cannabinoids ins Umfeld der Atemöffnungen (A) des menschlichen Körpers zu führen** eine **Rückhaltehaube** (1) umfasst, welches geeignet ist eine Effektivdosis mindestens eines Cannabinoids im Umfeld der Atemöffnungen (A) des menschlichen Körpers in einem Dampfgemisch zu erzeugen und zu erhalten.

6. Vorrichtung gemäss Anspruch 5, wobei die Rückhaltehaube durchlässig ausgestaltet ist für elektromagnetische Wellen, insbesondere für elektromagnetische Wellen in den Wellenlängenbereichen von zwischen 380 nm und 1100 nm.

7. Vorrichtung gemäss einem der Ansprüche 1 bis 6, umfassend einen **Speicherbehälter** für ein Verdampfungsmittel mit mindestens einem Cannabinoid, ein **Heizelement** zur Beheizung einer definierten Menge des Verdampfungsmittels, so dass ein Dampfgemisch mit einer definierten Menge des mindestens einen Cannabinoids entsteht.

8. Vorrichtung gemäss einem der Ansprüche 1 bis 7, umfassend mindestens einen **Ventilator** zur Erzeugung eines Luftstroms im Umfeld der Atemöffnungen (A) des menschlichen Körpers, insbesondere ein Luftstrom zur Extraktion des Dampfgemisch mit einer Effektivdosis mindestens eines Cannabinoids aus einer Rückhaltehaube (1).

9. Vorrichtung gemäss einem der Ansprüche 1 bis 8, umfassend mindestens einen **optoelektronischen Sensor,** insbesondere zur Ermittlung mindestens eines photoelektrisch messbaren physiologischen Parameters des menschlichen Körpers im Wirkbereich der Energie.

10. Vorrichtung gemäss einem der Ansprüche 1 bis 9, umfassend mindestens einen **bioelektronischen Sensor,** insbesondere zur Messung eines Widerstands.

11. Vorrichtung gemäss einem der Ansprüche 1 bis 10, wobei das Mittel um das Dampfgemisch mit einer Effektivdosis mindestens eines Cannabinoids ins Umfeld der Atemöffnungen (A) des menschlichen Körpers zu führen eine Einlassöffnung umfasst, welche ein ausserhalb einer Rückhaltehaube erzeugtes Dampfgemisch in diese leitet.

12. Vorrichtung gemäss einem der Ansprüche 1 bis 11, wobei das Mittel um das Dampfgemisch mit einer Effektivdosis mindestens eines Cannabinoids ins Umfeld der Atemöffnungen (A) des menschlichen Körpers zu führen eine Auslassöffnung umfasst, insbesondere eine mit einer Ansaugvorrichtung verbundene Auslassöffnung.

## Claims

1. Device for exposing a human body to energy, comprising:
a. A contact surface (2) that defines an exposure area in which a human body can be positioned within the coverage area of the energy;
b. At least one means of emitting the energy essentially directed at the exposure area, and
at least one means of generating a vapour mixture comprising at least one cannabinoid, and
at least one means of conveying the vapour mixture comprising an effective dose of at least one cannabinoid to the area surrounding the openings to the respiratory system (A) of the human body.

2. Device according to Claim 1, wherein the means of emitting the energy essentially directed at the exposure area is designed to emit electromagnetic energy, in particular radiation energy.

3. Device according to Claim 1, wherein the means of emitting the energy essentially directed at the exposure area is designed to emit kinetic energy, in particular a directed jet of fluid.

4. Device according to any of Claims 1 to 3, wherein the means of generating a vapour mixture is a vaporizer.

5. Device according to any of Claims 1 to 4, wherein the means of conveying a vapour mixture comprising an effective dose of at least one cannabinoid to the area surrounding the openings to the respiratory system (A) of the human body includes a retaining hood (1) that is suitable for the generation in a vapour mixture of an effective dose of at least one cannabinoid in the area surrounding the openings to the respiratory system (A) of the human body and the maintenance thereof.

6. Device according to Claim 5, wherein the retaining hood is designed to be transparent to electromagnetic waves, in particular to electromagnetic waves in the wavelength regions between 380 nm and 1100 nm.

7. Device according to any of Claims 1 to 6, including a storage tank for a vaporization agent comprising at least one cannabinoid and a heating element for heating a defined amount of a vaporization agent such that a vapour mixture comprising a defined amount of the at least one cannabinoid forms.

8. Device according to any of Claims 1 to 7, including at least one fan for generating an air flow in the area surrounding the openings to the respiratory system (A) of the human body, in particular an air flow for extraction of the vapour mixture comprising an effective dose of at least one cannabinoid from a retaining hood (1).

9. Device according to any of Claims 1 to 8, including at least one optoelectronic sensor, in particular for the determination of at least one photoelectrically measurable physiological parameter of the human body in the coverage area of the energy.

10. Device according to any of Claims 1 to 9, including at least one bioelectronic sensor, in particular for the measurement of resistance.

11. Device according to any of Claims 1 to 10, wherein the means of conveying a vapour mixture comprising an effective dose of at least one cannabinoid to the area surrounding the openings to the respiratory system (A) of the human body includes an inlet opening that feeds vapour mixture generated outside a retaining hood into said hood.

12. Device according to any of Claims 1 to 11, wherein the means of conveying a vapour mixture comprising an effective dose of at least one cannabinoid to the area surrounding the openings to the respiratory system (A) of the human body includes an outlet opening, in particular one connected to a suction device.

## Revendications

1. Dispositif destiné à appliquer de l'énergie à un corps humain, comprenant :
a. une surface de contact (2) qui définit une zone d'application dans laquelle un corps humain peut être placé dans la zone effective de l'énergie ;
b. au moins un moyen pour délivrer l'énergie dirigée essentiellement vers la zone d'application, et
au moins un moyen pour générer un mélange de vapeur comprenant au moins un cannabinoïde, et
au moins un moyen pour guider le mélange de vapeur contenant une dose efficace d'au moins un cannabinoïde vers l'environnement des orifices respiratoires (A) du corps humain.

2. Dispositif selon la revendication 1, dans lequel le moyen destiné à délivrer l'énergie dirigée essentiellement vers la zone d'application est conçu pour délivrer de l'énergie électromagnétique, en particulier de l'énergie rayonnante.

3. Dispositif selon la revendication 1, dans lequel le moyen destiné à délivrer l'énergie dirigée essentiellement vers la zone d'application est conçu pour délivrer de l'énergie cinétique, en particulier pour délivrer un jet de fluide dirigé.

4. Dispositif selon l'une des revendications 1 à 3, dans lequel le moyen destiné à générer un mélange de vapeur comprend un vaporisateur.

5. Dispositif selon l'une des revendications 1 à 4, dans lequel le moyen pour guider le mélange de vapeur contenant une dose efficace d'au moins un cannabinoïde vers l'environnement des orifices respiratoires (A) du corps humain comprend une housse de retenue (1) qui est adaptée pour générer et maintenir dans un mélange de vapeur une dose efficace d'au moins un cannabinoïde dans l'environnement des orifices respiratoires (A) du corps humain.

6. Dispositif selon la revendication 5, dans lequel la housse de retenue est conçue pour être perméable aux ondes électromagnétiques, en particulier aux ondes électromagnétiques dans les plages de longueurs d'onde comprises entre 380 nm et 1 100 nm.

7. Dispositif selon l'une des revendications 1 à 6, comprenant un réservoir de stockage pour un agent de vaporisation contenant au moins un cannabinoïde, un élément chauffant pour chauffer une quantité définie de l'agent de vaporisation, de manière à obtenir un mélange de vapeur contenant une quantité définie d'au moins un cannabinoïde.

8. Dispositif selon l'une des revendications 1 à 7, comprenant au moins un ventilateur pour générer un flux d'air dans l'environnement des orifices respiratoires (A) du corps humain, en particulier un flux d'air pour extraire le mélange de vapeur contenant une dose efficace d'au moins un cannabinoïde d'une housse de retenue (1).

9. Dispositif selon l'une des revendications 1 à 8, comprenant au moins un capteur optoélectronique, en particulier pour déterminer au moins un paramètre physiologique du corps humain mesurable par voie photoélectrique dans la zone effective de l'énergie.

10. Dispositif selon l'une des revendications 1 à 9, comprenant au moins un capteur bioélectronique, en particulier pour mesurer une résistance.

11. Dispositif selon l'une des revendications 1 à 10, dans lequel le moyen pour guider le mélange de vapeur contenant une dose efficace d'au moins un cannabinoïde vers l'environnement des orifices respiratoires (A) du corps humain comprend un orifice d'entrée qui dirige un mélange de vapeur généré à l'extérieur d'une housse de retenue vers celle-ci.

12. Dispositif selon l'une des revendications 1 à 11, dans lequel le moyen pour guider le mélange de vapeur contenant une dose efficace d'au moins un cannabinoïde vers l'environnement des orifices respiratoires (A) du corps humain comprend un orifice de sortie, en particulier un orifice de sortie relié à un dispositif d'aspiration.
